# EUROPEAN PATENT SPECIFICATION

(11) **EP 4 070 822 B1**
(45) Date of publication and mention of the grant of the patent: **12.06.2024**
(21) Application number: 22167225.6
(22) Date of filing: 07.04.2022
(51) Int. Cl.: A61L 9/22

(54) **AIR SANITIZATION APPARATUS AND OPERATING METHOD THEREOF**
LUFTREINIGUNGSGERÄT UND BETRIEBSVERFAHREN DAVON
APPAREIL D'ASSAINISSEMENT DE L'AIR ET SON PROCÉDÉ DE FONCTIONNEMENT

(30) Priority: 09.04.2021 IT 202100008903
(43) Date of publication of application: 12.10.2022
(73) Proprietor: Jonix S.p.A., 35020 Tribano (PD) (IT)
(72) Inventor: MANTOVAN, Mauro, 35020 TRIBANO (PD) (IT); CECCHI, Antonio, 35020 TRIBANO (PD) (IT)
(74) Representative: Studio Torta S.p.A.

(56) References cited:
- CN-A- 111 520 834
- CN-A- 111 940 411
- US-A1- 2008 170 971
- US-A1- 2010 247 389
- US-A1- 2017 056 543
- US-B2- 11 596 707

## Description

### FIELD OF THE ART

The present invention relates to an air sanitization apparatus and to the operating method thereof.

In particular, the present invention concerns the use of a sanitization apparatus designed to sanitize/purify the air from contaminating particles such as viruses and bacteria; the following discussion will make explicit reference thereto without losing generality.

### PRIOR ART

As known, some sanitization apparatuses are provided with an ionizer module arranged in an internal chamber communicating with the environment via an outlet, and one or more filters, which are arranged along the air sanitization path so as to filter the air before it passes through the ionizer module.

Some filters, usually those arranged immediately upstream of the ionizer module, are designed to filter out particles having a size smaller than 0.3 10⁻⁶ m.

A technical problem of the above-described sanitization apparatuses is that during maintenance and/or replacement operations of these filters, the maintenance technician is exposed to a risk of being contaminated by viruses or bacteria retained and accumulated in the filter.

In fact, both bacteria and viruses stay alive for a non-negligible amount of time when exposed to an ambient temperature of around 20°C.

Therefore, during the filter replacement operation, widespread contamination of viruses and bacteria can occur both involving the maintenance technician and the environment where the operation takes place.

In addition, over time, a continuous process of "mechanically" entraining a non-negligible percentage of viruses and bacteria present in the filter outside thereof and spreading them into the apparatus can take place, thereby causing contamination of some of the internal surfaces thereof. Sanitization apparatuses are also disclose in CN111940411A and CN111520834A.

### DISCLOSURE OF THE INVENTION

Aim of the present invention is to provide an air sanitization apparatus which is able to overcome the above-described technical problem.

According to the present invention, an air sanitization apparatus and an operating method thereof are provided as claimed by the respective appended Claims.

The Claims describe preferred embodiments of the present invention forming an integral part of the present description.

### BRIEF DESCRIPTION OF THE DRAWINGS

The invention is hereinafter described with reference to the accompanying drawings, wherein:
- Figure 1 is a perspective view, with parts removed for clarity, of an air sanitization apparatus according to the present invention,

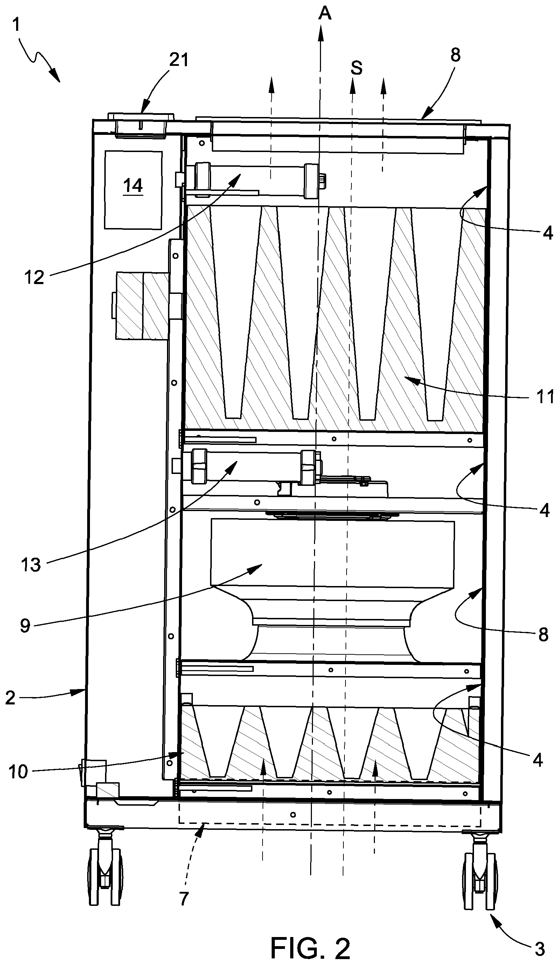

- Figure 2 is a front elevation view with parts removed for clarity of the air sanitization apparatus according to the present invention.

### PREFERRED EMBODIMENTS OF THE INVENTION

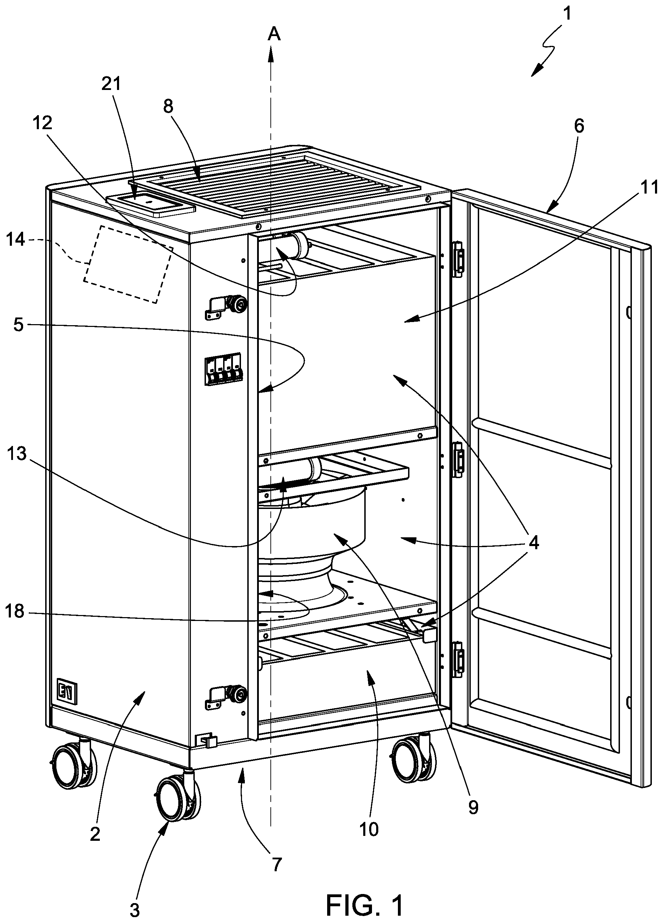

With reference to Figure 1, number 1 globally denotes an air sanitization apparatus designed to sanitize/purify the air present in an environment from contaminating substances and/or particles. Contaminating particles may comprise bacteria and/or viruses. Bacteria inactivated/killed by the apparatus 1 may comprise for example Escherichia coli, Staphylococcus epidermidis, Listeria monocytogenes, Legionella, or the like. Viruses inactivated/killed by the apparatus 1 may comprise, for example, chicken pox, measles, influenza, Sars-Cov, or the like.

It must be understood that the contaminating substances/particles may also comprise: volatile organic components or VOCs (based on Ethyl Acetate, Butyl Acetate, Toluene, Xylene or the like) and/or fungi (e.g. Aspergillus Brasiliensis, Candida Albicans or the like) and/or fine dust (PM2.5, PM10, etc.).

With reference to Figure 1, the air sanitization apparatus may comprise a closed container or boxed frame 2. The boxed frame 2 can be provided at the bottom with ground supporting wheels 3. The boxed frame 2 may include an internal chamber 4. The internal chamber 4 is accessible from the outside through a side opening 5. The opening 5 is designed to be closed by a door 6. The door 6 can be hinged with one vertical side on the boxed frame 2 so as to be movable (rotate) between an open position (shown in Figure 1) and a closed position.

According to a possible exemplary embodiment, the boxed frame 2 may be made of metal, have an approximately parallelepiped shape, and in the example shown has a preferably vertical reference axis A. When the door 6 is in the closed position, it delimits the internal chamber 4 with the internal walls of the boxed frame 2.

In addition to the opening 5 closed by the door 6, the internal chamber 4 communicates with the external environment also through at least one inlet 7 and at least one outlet 8. In the example shown, the inlet 7 may comprise a through opening obtained in the base or bottom wall of the boxed frame 2 transversal to the axis A.

The outlet 8 may comprise a through opening obtained in the upper wall of the boxed frame 2 transversal to the axis A.

The openings forming the inlet 7 and outlet 8 can be engaged by corresponding suction and delivery grids (with adjustable fins) respectively.

The sanitization apparatus 1 also comprises a fan assembly 9. The fan assembly 9 is arranged in the internal chamber 4. The fan assembly 9 may comprise at least one fan (not shown) having a preferably vertical rotation axis, driven to rotate by an electric motor (not shown). The fan may be, for example, a radial fan. The fan assembly 9 is designed to be operated so that the rotating fan sucks air from the environment through the inlet 7, conveys the air through the internal chamber 4 along a sanitization path S, and supplies the air to the environment through the outlet 8.

The sanitization apparatus 1 further comprises an air filtering device 10 (first filtering device), which is arranged in the internal chamber 4 upstream of the fan assembly 9 along said sanitization path S.

According to a preferred exemplary embodiment, the air filtering device 10 may comprise at least one coarse filter. In the example shown, the coarse filter is arranged below the fan assembly 9. The coarse filter may comprise a prefilter (e.g. a so-called G4 filter). In the example shown, the air filtering device 10 is arranged in a lower intermediate position of the internal chamber 4 so as to filter the air sucked through the inlet 7.

The sanitization apparatus 1 further comprises an air filtering device 11 (second filtering device), which is arranged in the internal chamber 4 downstream of the fan assembly 9 along the sanitization path S. According to a possible embodiment, the air filtering device 11 may comprise a fine particulate filter arranged in the internal chamber 4 above the fan assembly 9. In the example shown, the fine particle filter is structured to filter/retain particles having a size lower than 3*10⁻⁶ m (e.g. Class F9 or HEPA filter).

The sanitization apparatus 1 further comprises two non-thermal plasma generator ionizer devices 12 and 13 arranged in the internal chamber 4 to ionize the air therein.

A non-thermal plasma generator ionizer device refers to a reactive species generator by NTP technology (NTP - acronym for Non Thermal Plasma), which is designed to generate a gaseous mixture of air with ROS reactive species (acronym for Reactive Oxygen Species) and RNS reactive species (acronym for Reactive Nitrogen Species), hereinafter referred to more simply as NTP gas mixture.

Preferably, the non-thermal plasma generator ionizer device 12 (first non-thermal plasma generator ionizer device) is arranged in the internal chamber 4 between the filtering device 11 and the outlet 8.

The non-thermal plasma generator ionizer device 13 (second non-thermal plasma generator ionizer device) is arranged in the internal chamber 4 between the filtering device 10 and the filtering device 11.

In the example shown, the filtering device 10 and the filtering device 11 form relative horizontal walls or partitions which delimit in the internal chamber 4 with the vertical walls of the boxed frame 2, a compartment 18.

According to a possible embodiment, the non-thermal plasma generator ionizer device 13 is conveniently arranged approximately above the fan assembly 9 in a position adjacent to, immediately close to, the filter device 11.

According to a preferred embodiment, the non-thermal plasma generator ioniser devices 13 and 12 comprise one or more NTP generator ionizer modules (NTP - acronym for Non Thermal Plasma). Conveniently, the NTP generator ionizer modules of the non-thermal plasma generator ionizer devices 13 and 12 may comprise a bipolar ionizing tube or cylindrical capacitor. The bipolar ionizing tube can be structured to generate an electric field causing a corona effect when it is supplied with a *"*high voltage*"*, generally between 1500 and 4000 volts, so as to ionize the air around it. The ionized air is then appropriately *"*mixed*"* with the air to be sanitized to remove contaminating particles therefrom.

The sanitization apparatus 1 further comprises an electronic control unit 14 designed to control the operation of the sanitization apparatus 1. According to the present invention the electronic control unit 14 is configured to implement a filter sanitization process, wherein it controls the non-thermal plasma generator ionizer device 13 to generate a high predetermined quantity of NTP mixture in the internal chamber 4 in correspondence to at least the second filtering device 11 to sanitize it.

The Applicant has found that placing the non-thermal plasma generator ionizer device 13 in correspondence to the filter device 11 and selectively activating (switching on) the ionizer device 13 results in sanitization of the filtering device 11 within the apparatus 1.

A technical effect achieved is to reduce the cost of sanitizing the filter. The Applicant found that alternative embodiments for sanitizing filters involving the use of thermal sources would be more expensive and dangerous.

A further technical effect achieved is to reduce the risk of contaminating the subjects replacing the filters. A further technical effect achieved is to reduce the risk of contaminating internal parts of the apparatus 1 caused by living micro-organisms retained by the filter.

According to the invention, during the implementation of the filter sanitization process, the electronic control unit 14 selectively commands: the switching off of the fan assembly 9 in order to interrupt the flow of air passing through the filtering devices 10 and 11 in the internal chamber 4, and the switching on of the non-thermal plasma generator ionizer device 13 for a predetermined sanitization time interval associated with the first predetermined quantity of NTP mixture to be generated in the internal chamber 4.

The switching off of the fan assembly 9 has the technical effect of reducing the time taken to reach the first predetermined quantity of NTP mixture in the compartment 18 housing the filtering device 11.

Preferably, during the implementation of the filter sanitization process, the electronic control unit 14 commands the switching on of the fan assembly 9 when the first predetermined quantity of NTP mixture is reached.

The switching on of the fan assembly 9, when the first predetermined quantity of NTP mixture is reached, generates a high concentration flow of NTP mixture which passes through the filtering device 11 determining the sanitization thereof.

Preferably, during the implementation of the filter sanitization process, the electronic control unit 14 selectively commands the switching off of the ionizer device 13, and the switching on of the fan assembly 9 so as to reduce the quantity of NTP mixture in the internal chamber 4 from the first predetermined quantity (and the related and unavoidable Ozone by-productions) to a second predetermined quantity associated with a total safety condition for the users (people).

Preferably, the electronic control unit 14 may automatically activate the filter sanitization process in response to a command signal. The command signal can be generated, for example, by a user controller 21 present on the boxed frame 2. The user controller 21 may comprise a push button and/or a touch (sensitive) screen or the like. The touch screen defines a graphical interface that displays user selectable commands and/or information regarding the operation of the apparatus 1.

According to a further embodiment the command signal can be provided when contamination of the filtering device 11 is detected. For example, the electronic control unit 14 may detect that the filtering device 11 is contaminated on the basis of operating parameters of the apparatus. The operating parameters may comprise, for example, a value which is indicative of the quantity of air filtered, such as the number of activations of the apparatus 1 and/or the operating time and/or the quantity of air emitted or the like calculated since the last sanitization and/or since the last filter replacement. In addition and/or in alternative, according to a possible embodiment the electronic control unit 14 may activate the filter sanitization process on the basis of an operation time interval of the apparatus. For example, the electronic control unit 14 may activate the filter sanitization process when the operating time interval calculated from the previous sanitization and/or the last filter replacement reaches a predetermined threshold associated with a contamination condition of the filtering device 11.

Preferably, the electronic control unit 14 may generate an alarm message when it detects that the filtering device 11 is contaminated. Preferably an alarm message may be communicated to the user via the interface device 21.

In use, the operation method of the apparatus 1 comprises: receiving the command signal indicative of the contamination condition of the filtering device 11, preferably notifying to the user the presence of the contamination condition, and activating the implementation of the filter sanitization process. During the implementation of the filter sanitization process, the method provides switching off the fan assembly 9, switching off the non-thermal plasma generator ionizer device 12, and activating/controlling the non-thermal plasma generator ionizer device 13.

The non-thermal plasma generator ionizer device 13 may be controlled in order to generate a high quantity of NTP mixture (and inevitable related species including Ozone) until the first predetermined quantity of NTP mixture is reached in the internal chamber 4 at the filter device 11.

When the first predetermined quantity of NTP mixture has been reached, the method may provide switching off the non-thermal plasma generator ionizer device 13 and switching on the fan assembly 9. The method provides pushing the NTP mixture through the filtering device 11 in order to sanitize it.

After sanitizing the filter device 11, the apparatus 1 may be adapted to end the filter sanitization process and implement a process for sanitizing the air in the environment. When implementing the process of sanitizing the air in the environment, the method can implement the steps of: switching on the non-thermal plasma generator ionizer device 12 and the fan assembly 9. Air is sucked in through the inlet 7, filtered by the filtering device 10, filtered by filtering device 11, sanitized by the non-thermal plasma generator ionizer device 12 and supplied to the environment (as sanitized) through the outlet 8.

The apparatus described above is advantageous in that it is able to automatically sanitize the filtering device without any intervention by operators and reduces the contagion of the latter by contaminating particles during the filtering device replacement operation.

## Claims

1. Air sanitization apparatus (1) for sanitizing the air in an environment comprising:
- a boxed frame (2) provided with an internal chamber (4) communicating with the external environment through at least one inlet (7) and at least one outlet (8),
- fan means (9) which are arranged in said internal chamber (4) and are designed to be operated in order to: suck the air to be sanitized from the environment through the inlet (7), convey the air to be sanitized through the internal chamber (4) along a sanitizing path (S), and supplying the sanitized air into the environment through the outlet (8),
- a first filtering device (10), which is arranged in said internal chamber (4) upstream of the fan means (9) along said sanitizing path (S),
- a second filtering device (11), which is arranged in said internal chamber (4) downstream of said fan means (9) along said sanitization path (S),
- a first non-thermal plasma generator ionizer device (12), which is designed to generate an NTP mixture in said internal chamber (4) between the second filtering device (11) and said outlet (8), and **characterised in that**,
it further comprises:
- a second non-thermal plasma generator ionizer device (13) which is arranged in said internal chamber (4) between the first filtering device (10) and the second filtering device (11) and is designed to generate an NTP mixture between them,
- an electronic control unit (14), which is configured to implement a filter sanitization process, wherein it controls the second non-thermal plasma generator ionizer device (13) to generate a first predetermined quantity of NTP mixture in the internal chamber (4) in correspondence to at least the second filtering device (11) to sanitize it, wherein during the implementation of said filter sanitization process, said electronic control unit (14) is configured to selectively command:
the switching off of said fan means (9) in order to interrupt the flow of air passing through said filtering device (10) and second filtering device (11) in said internal chamber (4),
the switching on of the second non-thermal plasma generator ionizer device (13) for a predetermined time interval in order to generate said first predetermined quantity of NTP mixture.

2. Apparatus according to claim 1, wherein during the implementation of said filter sanitization process, said electronic control unit (14) is configured to control the switching on of said fan means (9) when said first predetermined quantity of NTP mixture is reached.

3. Apparatus according to claim 2, in which during the implementation of said filter sanitization process, said electronic control unit (14) is configured to selectively control:
the switching off of said second non-thermal plasma generator ionizer device (13),
the switching on of said fan means (9) for reducing the quantity of NTP mixture in the internal chamber (4) from said first predetermined quantity to a second predetermined quantity lower than said first quantity and associated with a prefixed concentration of ozone.

4. Apparatus according to claim 3, wherein during the implementation of said filter sanitization process, said electronic control unit (14) commands the switching off of said first non-thermal plasma generator ionizer device (12).

5. Apparatus according to any one of the preceding claims, wherein said electronic control unit (14) is configured to automatically implement said filter sanitization process.

6. Apparatus according to any one of the preceding claims, wherein said electronic control unit (14) is configured to implement said filter sanitization process, in response to a command given by a user.

7. Apparatus according to any one of the preceding claims, wherein said first (12) and second non-thermal plasma generator ionizer device (13) comprise bipolar ionizing tubes for generating electric fields designed to cause a corona effect in the air present in said chamber internal (4) .

8. Apparatus according to any one of the preceding claims, wherein said second filtering device (11) is structured to filter particles having dimensions less than or equal to about 3 * 10-6 m.

9. Method of operation of an air sanitization apparatus (1) in an environment including:
- a boxed frame (2) comprising an internal chamber (4) communicating with the external environment through at least one inlet (7) and at least one outlet (8),
- fan means (9) which are arranged in said internal chamber (4) and are designed to be operated in order to: suck the air to be sanitized from the environment through the inlet (7), convey the air to be sanitized through the internal chamber (4) along a sanitizing path (S), and supplying the sanitized air into the environment through the outlet (8),
- a first filtering device (10) which is arranged in said internal chamber (4) upstream of the fan means (9) along said sanitizing path (S),
- a second filtering device (11) which is arranged in said internal chamber (4) downstream of said fan means (9) along said sanitizing path (S),
- a first non-thermal plasma generator ionizer device (12) which is arranged in said internal chamber (4) between the second filtering device (11) and said outlet (8) and is able to generate an NTP mixture,
- a second non-thermal plasma generator ionizer device (13) which is arranged in said internal chamber (4) between the first filtering device (10) and the second filtering device (11) and is able to generate an NTP mixture between them,
selectively control by means of an electronic control unit (14) the second non-thermal plasma generator ionizer device (13) to generate a first predetermined quantity of NTP mixture in the internal chamber (4) at least at the second filtering device (11) so as to sanitize it, and
during the implementation of said filter sanitization process, selectively command by means of said electronic control unit (14):
the switching off of said fan means (9) in order to interrupt the flow of air passing through said filtering device (10) and second filtering device (11) in said internal chamber (4),
the switching on of the second non-thermal plasma generator ionizer device (13) for a predetermined time interval in order to generate said first predetermined quantity of NTP mixture.

## Patentansprüche

1. Luftreinigungsapparatur (1) zum Reinigen der Luft in einer Umgebung, die aufweist:
einen Einbaurahmen (2), der mit einer Innenkammer (4) versehen ist, die über mindestens einen Einlass (7) und mindestens einen Auslass (8) mit der Au-ßenumgebung kommuniziert,
eine Lüftereinrichtung (9), die in der Innenkammer (4) angeordnet und so gestaltet ist, dass sie betrieben wird, um: die zu reinigende Luft über den Einlass (7) aus der Umgebung anzusaugen, die zu reinigende Luft entlang eines Reinigungswegs (S) durch die Innenkammer (4) hindurch zu transportieren und die gereinigte Luft über den Auslass (8) der Umgebung zuzuführen,
eine erste Filtervorrichtung (10), die in der Innenkammer (4) stromaufwärts von der Lüftereinrichtung (9) entlang des Reinigungswegs (S) angeordnet ist,
eine zweite Filtervorrichtung (11), die in der Innenkammer (4) stromabwärts von der Lüftereinrichtung (9) entlang des Reinigungswegs (S) angeordnet ist,
eine erste nichtthermische Plasma-Erzeuger-Ionisator-Vorrichtung (12), die so gestaltet ist, dass sie ein NTP-Gemisch in der Innenkammer (4) zwischen der zweiten Filtervorrichtung (11) und dem Auslass (8) erzeugt, und
**dadurch gekennzeichnet, dass** sie ferner aufweist:
eine zweite nichtthermische Plasma-Erzeuger-Ionisator-Vorrichtung (13), die in der Innenkammer (4) zwischen der ersten Filtervorrichtung (10) und der zweiten Filtervorrichtung (11) angeordnet und so gestaltet ist, dass sie ein NTP-Gemisch zwischen ihnen erzeugt,
eine elektronische Steuereinheit (14), die so konfiguriert ist, dass sie einen Filterreinigungsvorgang implementiert, wobei sie die zweite nichtthermische Plasma-Erzeuger-Ionisator-Vorrichtung (13) steuert, um eine erste vorbestimmte Menge von NTP-Gemisch in der Innenkammer (4) in Entsprechung zu mindestens der zweiten Filtervorrichtung (11) erzeugen, um sie zu reinigen, wobei während der Implementierung des Filterreinigungsvorgangs die elektronische Steuereinheit (14) so konfiguriert ist, dass sie selektiv befiehlt:
das Ausschalten der Lüftereinrichtung (9), um die Luftströmung zu unterbrechen, die die Filtervorrichtung (10) und die zweite Filtervorrichtung (11) in der Innenkammer (4) durchläuft,
das Einschalten der zweiten nichtthermischen Plasma-Erzeuger-Ionisator-Vorrichtung (13) für ein vorbestimmtes Zeitintervall, um die erste vorbestimmte Menge von NTP-Gemisch zu erzeugen.

2. Apparatur nach Anspruch 1, wobei während der Implementierung des Filterreinigungsvorgangs die elektronische Steuereinheit (14) so konfiguriert ist, dass sie das Einschalten der Lüftereinrichtung (9) steuert, wenn die erste vorbestimmte Menge von NTP-Gemisch erreicht ist.

3. Apparatur nach Anspruch 2, wobei während der Implementierung des Filterreinigungsvorgangs die elektronische Steuereinheit (14) so konfiguriert ist, dass sie selektiv steuert:
das Ausschalten der zweiten nichtthermischen Plasma-Erzeuger-Ionisator-Vorrichtung (13),
das Einschalten der Lüftereinrichtung (9) zur Reduzierung der Menge von NTP-Gemisch in der Innenkammer (4) von der ersten vorbestimmten Menge auf eine zweite vorbestimmte Menge, die geringer als die erste vorbestimmte Menge und einer vorab festgelegten Ozonkonzentration zugeordnet ist.

4. Apparatur nach Anspruch 3, wobei während der Implementierung des Filterreinigungsvorgangs die elektronische Steuereinheit (14) das Ausschalten der ersten nichtthermischen Plasma-Erzeuger-Ionisator-Vorrichtung (12) befielt.

5. Apparatur nach einem der vorstehenden Ansprüche, wobei die elektronische Steuereinheit (14) so konfiguriert ist, dass sie den Filterreinigungsvorgang automatisch implementiert.

6. Apparatur nach einem der vorstehenden Ansprüche, wobei die elektronische Steuereinheit (14) so konfiguriert ist, dass sie den Filterreinigungsvorgang als Reaktion auf einen von einem Benutzer erteilten Befehl implementiert.

7. Apparatur nach einem der vorstehenden Ansprüche, wobei die erste (12) und zweite nichtthermische Plasma-Erzeuger-Ionisator-Vorrichtung (13) bipolare Ionisationsröhren zur Erzeugung elektrischer Felder aufweisen, die so gestaltet sind, dass sie einen Koronaeffekt in der Luft bewirken, die in der Innenkammer (4) vorhanden ist.

8. Apparatur nach einem der vorstehenden Ansprüche, wobei die zweite Filtervorrichtung (11) so strukturiert ist, dass sie Teilchen mit Abmessungen von höchstens etwa 3 * 10⁻⁶ m filtert.

9. Verfahren zum Betrieb einer Luftreinigungsapparatur (1) in einer Umgebung, die aufweist:
einen Einbaurahmen (2), der eine Innenkammer (4) aufweist, die über mindestens einen Einlass (7) und mindestens einen Auslass (8) mit der Außenumgebung kommuniziert,
eine Lüftereinrichtung (9), die in der Innenkammer (4) angeordnet und so gestaltet ist, dass sie betrieben wird, um: die zu reinigende Luft über den Einlass (7) aus der Umgebung anzusaugen, die zu reinigende Luft entlang eines Reinigungswegs (S) durch die Innenkammer (4) hindurch zu transportieren und die gereinigte Luft über den Auslass (8) der Umgebung zuzuführen,
eine erste Filtervorrichtung (10), die in der Innenkammer (4) stromaufwärts von der Lüftereinrichtung (9) entlang des Reinigungswegs (S) angeordnet ist,
eine zweite Filtervorrichtung (11), die in der Innenkammer (4) stromabwärts von der Lüftereinrichtung (9) entlang des Reinigungswegs (S) angeordnet ist,
eine erste nichtthermische Plasma-Erzeuger-Ionisator-Vorrichtung (12), die in der Innenkammer (4) zwischen der zweiten Filtervorrichtung (11) und dem Auslass (8) angeordnet ist und ein NTP-Gemisch erzeugen kann,
eine zweite nichtthermische Plasma-Erzeuger-Ionisator-Vorrichtung (13), die in der Innenkammer (4) zwischen der ersten Filtervorrichtung (10) und der zweiten Filtervorrichtung (11) angeordnet ist und ein NTP-Gemisch zwischen ihnen erzeugen kann,
mit Hilfe einer elektronischen Steuereinheit (14) erfolgendes selektives Steuern der zweiten nichtthermischen Plasma-Erzeuger-Ionisator-Vorrichtung (13), um eine erste vorbestimmte Menge von NTP-Gemisch in der Innenkammer (4) mindestens an der zweiten Filtervorrichtung (11) zu erzeugen, um sie zu reinigen, und
während der Implementierung des Filterreinigungsvorgangs mit Hilfe der elektronischen Steuereinheit (14) erfolgendes selektives Befehlen:
des Ausschaltens der Lüftereinrichtung (9), um die Luftströmung zu unterbrechen, die die Filtervorrichtung (10) und die zweite Filtervorrichtung (11) in der Innenkammer (4) durchläuft,
des Einschaltens der zweiten nichtthermischen Plasma-Erzeuger-Ionisator-Vorrichtung (13) für ein vorbestimmtes Zeitintervall, um die erste vorbestimmte Menge von NTP-Gemisch zu erzeugen.

## Revendications

1. Appareil d'assainissement de l'air (1) destiné à assainir l'air dans un environnement comprenant :
- un cadre caissonné (2) prévu avec une chambre intérieure (4) communiquant avec l'environnement extérieur via au moins une admission (7) et au moins une évacuation (8),
- des moyens de ventilation (9) qui sont agencés dans ladite chambre intérieure (4) et sont conçus pour être exploités afin de : aspirer l'air à assainir depuis l'environnement via l'admission (7), acheminer l'air à assainir à travers la chambre intérieure (4) le long d'un chemin d'assainissement (S) et amener l'air assaini jusque dans l'environnement via l'évacuation (8),
- un premier dispositif de filtrage (10), qui est agencé dans ladite chambre intérieure (4) en amont des moyens de ventilation (9) le long dudit chemin d'assainissement (S),
- un deuxième dispositif de filtrage (11), qui est agencé dans ladite chambre intérieure (4) en aval desdits moyens de ventilation (9) le long dudit chemin d'assainissement (S),
- un premier dispositif d'ionisateur de production de plasma non thermique (12), qui est conçu pour produire un mélange de plasma non thermique dans ladite chambre intérieure (4) entre le deuxième dispositif de filtrage (11) et ladite évacuation (8), et **caractérisé en ce qu'**il comprend en outre :
- un deuxième dispositif d'ionisateur de production de plasma non thermique (13), qui est agencé dans ladite chambre intérieure (4) entre le premier dispositif de filtrage (10) et le deuxième dispositif de filtrage (11) et est conçu pour produire un mélange de plasma non thermique entre eux,
- une unité de commande électronique (14), qui est configurée pour mettre en oeuvre un processus d'assainissement de filtre, dans lequel il commande le deuxième dispositif d'ionisateur de production de plasma non thermique (13) pour produire une première quantité prédéterminée de mélange de plasma non thermique dans la chambre intérieure (4) en correspondance avec au moins le deuxième dispositif de filtrage (11) afin de l'assainir, dans lequel pendant la mise en oeuvre dudit processus d'assainissement de filtre, ladite unité de commande électronique (14) est configurée pour ordonner de façon sélective :
l'extinction desdits moyens de ventilation (9) afin d'interrompre le flux d'air passant à travers lesdits dispositif de filtrage (10) et deuxième dispositif de filtrage (11) dans ladite chambre intérieure (4),
l'allumage du deuxième dispositif d'ionisateur de production de plasma non thermique (13) pendant un intervalle de temps prédéterminé afin de produire ladite première quantité prédéterminée de mélange de plasma non thermique.

2. Appareil selon la revendication 1, dans lequel pendant la mise en oeuvre dudit processus d'assainissement de filtre, ladite unité de commande électronique (14) est configurée pour commander l'allumage desdits moyens de ventilation (9) lorsque ladite première quantité prédéterminée de mélange de plasma non thermique est atteinte.

3. Appareil selon la revendication 2, dans lequel pendant la mise en oeuvre dudit processus d'assainissement de filtre, ladite unité de commande électronique (14) est configurée pour commander de façon sélective :
l'extinction dudit deuxième dispositif d'ionisateur de production de plasma non thermique (13),
l'allumage desdits moyens de ventilation (9) pour réduire la quantité de mélange de plasma non thermique dans la chambre intérieure (4), de ladite première quantité prédéterminée à une deuxième quantité prédéterminée inférieure à ladite première quantité et associée à une concentration prédéfinie d'ozone.

4. Appareil selon la revendication 3, dans lequel pendant la mise en oeuvre dudit processus d'assainissement de filtre, ladite unité de commande électronique (14) ordonne l'extinction dudit premier dispositif d'ionisateur de production de plasma non thermique (12).

5. Appareil selon l'une quelconque des revendications précédentes, dans lequel ladite unité de commande électronique (14) est configurée pour automatiquement mettre en oeuvre ledit processus d'assainissement de filtre.

6. Appareil selon l'une quelconque des revendications précédentes, dans lequel ladite unité de commande électronique (14) est configurée pour mettre en oeuvre ledit processus d'assainissement de filtre, en réponse à un ordre donné par un utilisateur.

7. Appareil selon l'une quelconque des revendications précédentes, dans lequel lesdits premier (12) et deuxième dispositifs d'ionisateur de production de plasma non thermique (13) comprennent des tubes ionisants bipolaires afin de produire des champs électriques conçus pour provoquer un effet de couronne dans l'air présent dans ladite chambre intérieure (4).

8. Appareil selon l'une quelconque des revendications précédentes, dans lequel ledit deuxième dispositif de filtrage (11) est structuré de façon à filtrer des particules présentant des dimensions inférieures ou égales à environ 3 * 10-6 m.

9. Procédé d'exploitation d'un appareil d'assainissement de l'air (1) dans un environnement incluant :
- un cadre caissonné (2) comprenant une chambre intérieure (4) communiquant avec l'environnement extérieur via au moins une admission (7) et au moins une évacuation (8),
- des moyens de ventilation (9) qui sont agencés dans ladite chambre intérieure (4) et sont conçus pour être exploités afin de : aspirer l'air à assainir depuis l'environnement via l'admission (7), acheminer l'air à assainir à travers la chambre intérieure (4) le long d'un chemin d'assainissement (S) et amener l'air assaini jusque dans l'environnement via l'évacuation (8),
- un premier dispositif de filtrage (10), qui est agencé dans ladite chambre intérieure (4) en amont des moyens de ventilation (9) le long dudit chemin d'assainissement (S),
- un deuxième dispositif de filtrage (11), qui est agencé dans ladite chambre intérieure (4) en aval desdits moyens de ventilation (9) le long dudit chemin d'assainissement (S),
- un premier dispositif d'ionisateur de production de plasma non thermique (12) qui est agencé dans ladite chambre intérieure (4) entre le deuxième dispositif de filtrage (11) et ladite évacuation (8), et est à même de produire un mélange de plasma non thermique,
- un deuxième dispositif d'ionisateur de production de plasma non thermique (13) qui est agencé dans ladite chambre intérieure (4) entre le premier dispositif de filtrage (10) et le deuxième dispositif de filtrage (11) et est à même de produire un mélange de plasma non thermique entre eux,
commander de façon sélective au moyen d'une unité de commande électronique (14) le deuxième dispositif d'ionisateur de production de plasma non thermique (13) afin de produire une première quantité prédéterminée de mélange de plasma non thermique dans la chambre intérieure (4) au moins au niveau du deuxième dispositif de filtrage (11) de façon à l'assainir, et
pendant la mise en oeuvre dudit processus d'assainissement de filtre, ordonner de façon sélective au moyen de ladite unité de commande électronique (14) :
l'extinction desdits moyens de ventilation (9) afin d'interrompre le flux d'air passant à travers lesdits dispositif de filtrage (10) et deuxième dispositif de filtrage (11) dans ladite chambre intérieure (4),
l'allumage du deuxième dispositif d'ionisateur de production de plasma non thermique (13) pendant un intervalle de temps prédéterminé afin de produire ladite première quantité prédéterminée de mélange de plasma non thermique.
